# EUROPEAN PATENT SPECIFICATION

(11) **EP 1 991 066 B1**
(45) Date of publication and mention of the grant of the patent: **15.09.2010**
(21) Application number: 07705759.4
(22) Date of filing: 01.02.2007
(51) Int. Cl.: A23L 1/00, A23L 1/22, B01J 13/02, C11D 3/37, C11D 3/50, C11D 11/02, A61K 8/11

(54) **PROCESS FOR THE PREPARATION OF POWDERS FROM SLURRIES OF FRAGRANCED AMINOPLAST CAPSULES**
VERFAHREN ZUR HERSTELLUNG VON PULVERN AUS AUFSCHLÄMMUNGEN VON PARFÜMIERTEN AMINOPLASTKAPSELN
PROCÉDÉ POUR LA PRÉPARATION DE POUDRES À PARTIR DE SUSPENSIONS ÉPAISSES DE CAPSULES D'AMINOPLASTE CONTENANT UN PARFUM

(30) Priority: 24.02.2006 WO PCT/IB2006/050593
(43) Date of publication of application: 19.11.2008
(73) Proprietor: Firmenich S.A., 1211 Geneva 8 (CH)
(72) Inventor: VERHOVNIK, Glenn, CH-1224 Chene-Bougeries (CH); NGUYEN, Van Cung, CH-1220 Les Avanchets (CH)
(74) Representative: Salvaterra-Garcia, Maria de Lurdes
(86) International application number: PCT/IB2007/050337
(87) International publication number: WO 2007/096790

(56) References cited:
- EP-A2- 0 415 273
- WO-A-03/043728
- WO-A-2004/016234
- WO-A-2005/123892

## Description

### Technical Field

The present invention relates to the perfume and consumer product industries. It concerns more particularly a process for producing powders out of slurries of aminoplast, and more particularly melamine-formaldehyde, capsules containing fragrances and other hydrophobic materials. The invention also relates to the powder products thus obtained, which have novel physical and chemical characteristics, and to the use of such powders in consumer products. The consumer products containing such powders of melamine-formaldehyde capsules are also an object of the invention.

### Background Art

The literature is rich in reports of the use of melamine-formaldehyde resins for the encapsulation of active substances and mentions in particular the potential use of such encapsulation systems in perfumery and cosmetic applications. Therefore, amino resin based capsules, also commonly designated as aminoplast capsules, are the subject of a variety of literature reports and patent applications relating to the perfumery and cosmetic industries. In practice, these polymers are capable of forming a protecting shell around the active ingredient that one wishes to protect, thus providing an encapsulation system characterised by its water-insolubility. The active ingredient protected by the capsule may be released through mechanical rupture of the microcapsules, which become brittle when dry.

Some of the typical examples of this type of applications can be found in the following patent documents, but the person skilled in the art will no doubt have knowledge of many other reported applications of fragranced aminoplast capsules in consumer products.

US 5,137,646 describes coated perfume particles for use in fabric softeners and antistatic agents and US 6,620,777 claims improved deposition of fragranced aminoplast microcapsules on fabric from liquid fabric softeners by adding cationic polymers.

US 4,145,184 claims granular and liquid laundry detergent compositions comprising fragranced microcapsules.

US 6.248,703 discloses soap bar and detergent bar compositions comprising melamine capsules that contain essential oils.

WO 03/089561 describes the use of fragranced microcapsules in fabric refresher aerosols.

The use of such fragranced aminoplast capsules in liquid formulations for household and cosmetic applications is also known for example from US Patent 5,188,754, assigned to Procter & Gamble, which describes detergent compositions containing perfumes in the form of friable microcapsules. US Patent 5,137,646, also to Procter & Gamble, describes the preparation and use of perfumed aminoplast particles which are stable in fluid compositions such as fabric softeners. However, this composition requires a two-step manufacturing process where the perfume is firstly solidified with a meltable polymer, followed by grinding of the solidified perfume and coating with the aminoplast resin.

The technical advantage of such friable aminoplast microcapsules is that they are water insoluble and resistant to heat. As a result, the fragrance or other hydrophobic substance remains fully encapsulated when capsules are dispersed in aqueous formulations and during laundering processes for example. The fragrance is mainly released by mechanical manipulation of the dry target surface such as skin, hair or fabric. A general range of encapsulate concentration, namely fragrance content, of the microcapsules slurry is between 1 and 50%, by weight, relative to the weight of the slurry, the latter also containing typically 4 to 20% by weight of encapsulating shell material in a free form, and the balance being water.

The general process for the preparation of aminoplast microcapsules containing encapsulated fragrances or other hydrophobic materials is also a well-known state of the art and is widely described in the patent literature. Early examples of the latter are represented by US Patents 3,016,308, 3,516,846 and 3,516,941, as well as US 4,396,670 to The Wiggins Teape Group Ltd., and many other more recent patent documents have addressed this subject.

One can also find general literature reviews of such methods as well as of the uses of the microcapsules obtained there-from. For the sake of example there will be cited here the publications K. Dietrich et al. (1989 and 1990), Amino resin microcapsules, Acta Polymerica 40(4), pgs. 243-251, 40(5), pgs. 325-331, 40(11), pgs. 683-690 and 41(2), pgs. 91-95. Another general review of interest is that of H. Y. Lee et al., J. Microencapsulation (2002), 19 (5), pages 559-569.

The person skilled in the art is also referred to recent documents such as International Patent Application WO 2006/018694, belonging to the present Applicant, as well as to US 6,024,943 to Quest International and WO 01/41915 to Microcapsules Technologies, and to the references generally cited in these documents.

The preferred encapsulating shell material is a polymeric shell, which is the reaction product of urea or melamine and an aldehyde, such as formaldehyde. When aqueous slurries of such microcapsules are dried, the water evaporates and the encapsulated fragrance is fully retained in the dried microcapsules.

The micro encapsulation principle is relatively simple. A thin polymer shell is created around droplets or particles of an active agent emulsified or dispersed in a carrier liquid. Preferred polymer materials for the microcapsules shell are those which are capable of polymerisation under acid pH from a water-soluble pre-polymer state. Such pre-polymers are made by reacting urea and formaldehyde in a formaldehyde urea molar ratio of from about 1.2:1 to 2.6:1. Thiourea, cyanuramide, guanidine, N-alkyl ureas, phenols, sulfonamides, anilines and amines can be included in small amounts as modifiers for the urea. These polymers formed from such pre-polymer materials, under acidic conditions, are water-insoluble and can provide the required capsule friability characteristics.

As described in US 4,233,178, microcapsules based on melamine/formaldehyde resins are preferred over those based on urea/formaldehyde resins, because the resulting capsules are more impermeable due to a higher degree of cross-linking of the film-forming resin.

Microcapsules made from the preferred melamine-formaldehyde shell materials can be made by the processes described in WO 01/51197, US 6,261,483, US 2003/0004226, US 4,406,816 and EP 0415273.

In all the known processes, a large part of the obtained capsule slurry is water, and it often desired to eliminate this water and to obtain a dry capsule concentrate.

Although in many cases the capsule slurry can be used, in certain applications, it is preferred to use such aminoplast capsules in a dry form because water disturbs the final structure or composition of the final product into which the microcapsules are incorporated. Examples are water-free liquid detergents and fabric softeners for example, namely those packaged in water soluble polyvinyl alcohol pouches, hair oils, body oils, hair and body sprays based on cyclomethicone, powder detergents and fabric conditioning sheets typically intended for use in tumble dryers. In this latter case, during the preparation of the dryer sheets water-insoluble esterquats are applied as a hot melt on a non-woven material. These waxes are incompatible with water and have a melting point that is higher than the boiling point of water. Incorporation of aqueous slurries of aminoplast capsules is therefore not possible under such conditions.

Another example of specific applications where melamine microcapsule solids are appropriate is the case of anhydrous liquid detergents which have the technical advantage that they can contain bleach and bleach activators but wherein the latter will react immediately in the presence of water, as described for example in US 4,800,035 or US 5,057,238, to Colgage Palmolive. It is therefore not convenient to add any aqueous emulsions or dispersions to such formulations.

Non aqueous fabric softeners can be formulated on a completely oil-based composition which is then stable in water soluble polyvinyl alcohol packages such as described in US 6,492,315, to Colgate Palmolive. In such water-free bases, it is difficult to incorporate aqueous dispersions of microcapsules because they would readily separate from the formulation and attack the water soluble packaging, possibly causing leaking of the liquid softener during storage.

In all such cases it is therefore required to incorporate the melamine-formaldehyde microcapsules in solid form and the present invention brings exactly an advantageous solution to this problem by providing a process for converting the aqueous slurries into free flowing powders via spray drying of the slurry under well-controlled conditions.

Although the concept of spray drying the slurries has been generally and briefly mentioned for example in US 20040071742 and in WO 2004/016234, no particular details or guidance to the specific process conditions used has been reported in the prior art. We have now surprisingly found that spray drying of the slurries must be carried out under specific processing parameters and using specific carrier materials in order to obtain a free flowing powder of the microcapsules enclosing the hydrophobic perfume or other material.

In fact, we have been able to establish that, under many current spray drying method conditions, encompassed by a general reference or citation of possible spray drying methods to be applied to such aminoplast capsule slurries, there are not in fact obtained free flowing powders but products wherein the microcapsules tend to agglomerate and deliver unstable materials upon storage.

This is a consequence of the fact that, as mentioned above, aminoplast microcapsules are generally prepared by interfacial polymerization in the presence of emulsifiers. These emulsifiers can be anionic thickeners such as carboxymethylcellulose, gum arabic, polyvinyl alcohol, or combinations of such polymers, with anionic surfactants such as sodium dodecyl sulfate. Such anionic thickeners are not incorporated into the capsule membrane and are still present in the final slurry.

In other approaches, emulsifiers that have no thickening properties are used and they are incorporated into the capsule membrane during the polymerization process. Such emulsifiers are sulfonated copolymers of acrylamide and acrylic acid as described in WO 01/51197. To avoid separation of capsules from the final slurry, thickening polymers need to be post-added, requiring a further processing step to obtain viable powders.

We have now found that, when aminoplast capsules are spray-dried, the thickening polymers strongly disturb the process. During the spray-drying process, the aminoplast capsules form sticky agglomerates and deposits on the inner walls of the spray-dryer because the melamine resin continues crosslinking under the high temperature conditions of the spray drying process. The presence of the thickening polymers in the aminoplast slurry thus promotes further agglomeration of the microcapsules amongst themselves and onto the reactor wall of the spray-dryer. Such deposits cannot be easily washed-off from the reactor walls and mechanical cleaning is required, increasing production time and cost. Moreover, the spray-dried powder is only obtained in small yields of maximum 30% and forms agglomerates and lumps that cannot be easily dispersed into liquid formulations. As a result, aminoplast microcapsules that are prepared according to the processes described in WO 98/28396 and US 5,188,753, for example, cannot in fact be efficiently spray-dried, because of the presence of the anionic emulsifying thickeners required for the encapsulation process.

The present invention provides a process which avoids such problems. The process of the invention allows spray drying of aminoplast microcapsule slurries under specific conditions described hereafter.

Moreover, the process of the invention dispenses with the need to add post-encapsulation thickening polymers to stabilize the aqueous slurry of capsules, thus avoiding their negative impact on the spray-drying process

We have now found that addition of de-tackifying colloids selected from modified polydextrose derivatives to aminoplast microcapsule slurries provides a temporary second coating around the microcapsules which inhibits further reaction of the aminoplast resin during the spray-drying.

Water-insoluble inorganic powders such as zeolites, bentonites or silica, can be considered as de-tackifying colloids, but a disadvantage of the use of such inorganic colloids is their strong thickening properties, rendering the microcapsule slurry difficult to spray-dry. In fact, usually such inorganic flowing agents are rather added to the dry powder after spray-drying in order to increase its flowability.

Other de-tackifying colloids are anionic organic polymers, like gum Arabic, or non-ionic organic polymers such as polyvinyl alcohol or alkyl modified cellulose derivatives, as previously cited. Typical examples are ethyl cellulose or hydroxypropyl cellulose. Hydrocarbons and sugars, such as sucrose, mannose, maltose, sorbitol, xylitol or copolymers of sucrose and dextrose known as refined polydextrose (Litesse^{®} from Danisco), or yet dehydrated glucose obtained from hydrolysis of starch (Glucidex^{®} 6 from Roquette), have also been used as de-tackifying agents.

However, due to their lack of emulsification properties, many of such products still allow some agglomeration of the microcapsules during spray drying and need to be added at fairly high quantities in order to reduce the sticky deposits of the capsules on the reactor walls of the spray-dryer to allow easy wash-off thereof with water.

It is thus that, although in WO 2004/016234 to Quest, spray-drying of aminoplast microcapsules in the presence of carboxymethylcellulose is described in example 7, no specific benefit of the use of this water-soluble coating could in fact be found with regard to this microcapsule agglomeration and wall depositing problem. Although the resulting powder is described in this document as being free-flowing, in our own experience such spray-dried powders were sticky and difficult to wash off from the spray-drier.

The present invention provides a process for the preparation of powders from aqueous aminoplast microcapsule slurries, via spray drying the latter using specific materials as spray drying carriers. The process of the invention allows full retention of the encapsulated hydrophobic material, namely fragrance, during the spray drying and makes it possible to improve the odor quality of the aminoplast microcapsules thus obtained.

### Disclosure of the Invention

The present invention provides a process for the preparation of powders of aminoplast, and more particularly melamine-formaldehyde, microcapules containing fragrances or other hydrophobic materials, wherein an aqueous slurry of aminoplast microcapsules, comprising a water soluble organic de-tackifying colloid having emulsifying properties, is spray-dried in a generally known manner. Prior to the spray-drying the pH of the slurry is adjusted to a value comprised between 4 and 6 by means of an acid.

According to a preferred embodiment of the invention, the aminoplast capsule slurry further contains a flame retardant agent. Suitable such agents are described in detail in International patent applications WO 03/043728 and PCT/IB2005/053089, belonging to the present Applicant. The use of short chain carboxylic acids, as described therein, is preferred.

The concentration of capsules in the aqueous slurries which are dried according to the process of the invention will be typically comprised between 0.5 and 50% by weight, relative to the total weight of aqueous suspension.

The dry capsules resulting from the process of the invention form a non-sticky powder that is easy to wash-off with hot water from the reactor walls of the spray-drying tower, does not have any amine smell (unlike what is the case with the dried aminoplast microcapsule products prior known) and is free flowing. Moreover, it easily disperses in aqueous and non-aqueous formulations of a variety of liquid consumer products without the help of any dispersing agents. In water, the powder disperses into the original particle size of the microcapsules without the formation of agglomerates, the particle size being fully retained during the spray-drying process.

We have now established that water soluble colloids as defined in claim 1 with emulsifying properties showed excellent de-tackifying performance during the spray-drying process of aminoplast capsules. Addition of such emulsifying colloids strongly increased the final yield of spray-dried powder and allowed easy cleaning of the spray-drier with water. In particular, addition of lipophilised cellulose such as dioctenylsuccinated starch (Capsul^{®} from National Starch), HI-CAP^{®} 100 (hydrogen octenylbutanedioate starch), or of emulsion stabilizing starch derivatives obtained from waxy maize (such as the commercial products of the type of Purity Gum 2000 from National Starch) to the aminoplast capsule slurry avoided agglomeration and lump formation of the spray-dried microcapsules and allowed easy dispersion of the dried capsules in the end-product liquid formulations into which they were incorporated.

Moreover, the above-mentioned preferred embodiment of the process of the invention is particularly useful for preparing powdered melamine-formaldehyde based microcapsules. This type of microcapsules typically exhale a strong amine smell. We have now been able to completely eliminate this malodor by reducing the pH of the slurry to pH = 5 with an acid such as citric acid, hydrochloric acid or sulfonic acid for example. Surprisingly, we have also established that the acid further improves the emulsification properties of the colloid, namely Capsul^{®}, thus improving the dispersibility of the microcapsule powder thus obtained in liquid formulations.

The emulsifying properties of such modified starch derivatives could be clearly seen in the resulting particle size distribution of the spray-dried capsules. When Capsul^{®} or Purity Gum 2000 were added to the capsule slurry, the spray-dried powder obtained showed a more homogeneous and smaller average particle size, compared to capsules spray-dried in the presence of Litesse^{®} Ultra or Glucidex^{®} 6, which were also tested but did not provide as good results, or required substantially higher amounts to be used to achieve similar results.

According to the invention, a modified starch of anionic character with emulsifying properties is preferred as the detackifying colloid. The modified starch is added to the slurry in an amount comprised between 1 and 10%, and more preferably between 1 and 3% by weight, relative to the weight of the slurry.

The modified starches appropriate for use according to the invention include hydrolyzed starch, acid thinned starch, starch esters of long chain hydrocarbons, starch acetates, starch octenyl succinate, and mixtures thereof.

The term "hydrolyzed starch" is meant to mean here oligosaccharide-type materials that are typically obtained by acid and/or enzymatic hydrolysis of starches, preferably corn starch. Suitable hydrolyzed starches include maltodextrins and corn syrup solids. Starch esters having a degree of substitution in the range of from about 0.01% to about 10.0% are also useful. The hydrocarbon part of the modifying ester should be from a C₅ to C₁₆ carbon chain.

Preferably, octenylsuccinate substituted waxy corn starches of various types shall be used, such as:
1) waxy starch: acid thinned and octenylsuccinate substituted;
2) blend of corn syrup solids: waxy starch, octenylsuccinate substituted, and dextrinized;
3) waxy starch: octenylsuccinate substituted and dextrinized;
4) blend of corn syrup solids or maltodextrins with waxy starch: acid thinned octenylsuccinate substituted, and then cooked and spray dried; and
5) waxy starch: acid thinned and octenylsuccinate substituted, then cooked and spray dried
can also be used in the present invention. Commercialized starches that are advantageously used in the context of the invention include the products sold under the tradenames Capsul^{®}, HiCap^{®} and Alcocap^{®}, as well as Purity Gum 2000, all from National Starch.

According to very advantageous embodiments of the process of the invention, there is further added to the microcapsule slurry a flame retardant agent which is intended to allow for reduction of the powder explosivity during the spray drying process and/or during storage thereof.

The flame retardant agents that are appropriate for use according to the invention include all the materials that have been cited in this capacity in the previously cited International patent applications, the contents of which are hereby included by reference. Examples of such ingredients are described in detail in these documents which further disclose the appropriate amounts thereof to be used in spray drying processes.

As the encapsulate in the microcapsules of the invention, there can be used a wide range of water- insoluble materials, amongst which there can be cited flavors and fragrances, antimicrobial or bacteriostatic agents, or yet malodor counteracting agents, and cosmetic/cosmaceutical or nutritional/nutraceutical ingredients. Following preferred embodiments of the invention, will be used fragrance compositions or ingredients, antibacterial or malodor counteracting agents.

Preferred embodiments resort to the use of fragrance ingredients or compositions. By fragrance ingredients or composition it is understood here an ingredient or mixture of ingredients capable of activation an olfactive receptor of a human or animal subject. These are typically mixtures of ingredients capable of imparting a pleasant odor to the end product into which the microcapsules are incorporated, and the skilled perfumer is able to create such mixtures as a function of the perfuming effect that it is desired to impart and the perfumer's own creative capabilities.

The nature of the fragrance contained in the capsules is therefore immaterial in the context of the invention, provided that it is compatible with the materials forming the capsules. It will be typically chosen as a function of the perfuming effect that is desired to achieve with the dispersion or consumer product of the invention, and it will be formulated according to current practices in the art of perfumery. It may consist of a perfume ingredient or a composition. These terms can define a variety of odorant materials of both natural and synthetic origin, currently used for the preparation of perfumed consumer products. They include single compounds or mixtures. Specific examples of such components may be found in the current literature, e.g. Perfume and Flavor Chemicals by S. Arctander 1969, Montclair, N.J. (USA). These substances are well known to the person skilled in the art of perfuming consumer products, i.e. of imparting an odor to a consumer product traditionally fragranced, or of modifying the odor of said consumer product.

Natural extracts can also be encapsulated into the system of the invention; these include e.g. citrus extracts such as lemon, orange, lime, grapefruit or mandarin oils, or essentials oils of plants, herbs and fruits, amongst other.

The prior art is rich in citations of such fragrance compositions and even in reports of allegedly optimised perfumes to be encapsulated, so as to provide stable microcapsules. General rules of perfume creation have been used for a while as evidenced by the contents of earlier patent literature of which the patent publications WO 01/41915 to Microcapsule Technologies, and WO 98/28398 and WO 02/74430 to Quest International are representative examples freely available to the perfumer. All these prior art reports are part of the general knowledge in the art and can be used by the skilled perfumer without particular difficulty and are thus naturally incorporated into the present disclosure by way of reference and citation. A more detailed description of the fragrance compositions that can be used according to the invention is thus not warranted here.

The amount of oil encapsulated in the powders obtained according to the invention varies in a wide range values, typically comprised between 1 and 90% by weight, more preferably between 20 and 80% by weight, relative to the weight of powder.

In addition to the fragranced capsules, the perfuming microcapsules obtained according to the invention may comprise optional ingredients such as antibacterial agents, cosmetic emollients, vitamins, cooling agents, softeners, lubricants, gloss enhancing agents or any other current active ingredient used in cosmetic or household applications, as long as the latter do not alter the capsules.

The invention also relates to the consumer products obtained by incorporation of the microcapsule powders obtained as described above in the final product. Examples are water-free liquid detergents and fabric softeners, namely those packaged in water soluble polyvinyl alcohol pouches, hair oils, body oils, hair and body sprays based on cyclomethicone, powder detergents and fabric conditioning sheets typically intended for use in tumble dryers.

The concentration in which the powder microcapsules of the invention can be added to these products are typically comprised between 0.1 and 10.0% by weight relative to the weight of the product. Such concentrations are given strictly by way of example since their value depends on the perfuming effect desired and on the nature of the product and can be easily adjusted accordingly.

The invention will now be described in further detail by way of the following examples.

### Example 1

### Preparation of melamine-formaldehyde microcapsule aqueous slurries

a) Fragranced melamine-formaldehyde capsules were prepared by the process described in US 5,162,486. The resulting microcapsule slurry contained 40% by weight of microcapsules, with 32% by weight of encapsulated fragrance. The encapsulated model fragrance POLYNESIA CP 147869 H (woody, floral; origin: Firmenich SA) had a flash point of 97°C.
   Such fragranced microcapsules are commercially available under the trade name PopScent^{®} from Firmenich SA.
   The capsule's content of the slurry was determined by analyzing the dry solids thereof using a microwave balance from Sartorius (Model MMA 30).
   The encapsulated fragrance content was determined by hydro-distillation.
   Particle size of the microcapsules in the slurry was around 2 to 3 microns, as determined via a Malvern particle size analyser.
   No thickening polymer was added to the slurry. To avoid separation of the microcapsules from the slurry, the dispersion was continuously agitated during the spray-drying process.
b) To the aqueous microcapsule dispersion obtained in a), there was added 0.2%, by weight, relative to the slurry weight, of Tylose^{®} 60'0000, available from Clariant. Tylose^{®} 60'000 is a water soluble, non-ionic thickening polymer (Methylhydroxyethyl Cellulose). The thickener stabilized the aqueous capsule dispersion and no continuous agitation was required in order to keep a homogeneous aqueous dispersion of the capsules.
c) To the aqueous microcapsule dispersion obtained in a), there was added 0.2%, by weight, relative to the slurry weight, of Blanose^{®} from Aqualon. Blanose^{®} is a water soluble, anionic thickening polymer (refined Carboxymethyl Cellulose). The thickener stabilized the aqueous capsule dispersion and no continuous agitation was required in order to keep a homogeneous aqueous dispersion of the capsules.
d) Fragranced melamine-formaldehyde capsules were prepared as described in a) using, as the encapsulated raw material, Limonene, which has a flash point of 47°C.

### Example 2

### Preparation of melamine-formaldehyde microcapsule powders

To 100 parts by weight of the slurry of aminoplast microcapsules from Example 1a) there were added 52 parts by weight of a 20% aqueous solution of different modified starches as summarized in Table I below. The blends were neutralized with citric acid (50% aqueous solution) until pH = 5.

The microcapsule aqueous dispersions thus obtained were then spray-dried at Büchi Spray Dryer Model B 290.

The temperature of the incoming air in the spray dryer was 185°C - 190°C, the temperature of the outcoming air 95°C - 105°C. A biphase spray nozzle was used with a nozzle diameter of 0.7 mm.

**Table I**

| | **SAMPLE / Parts by weight** | | | |
|---|---|---|---|---|
| **Ingredients** | **A** | **B** | **C** | **D** |
| Example 1a) microcapsule slurry (no colloid) | 100 | 100 | 100 | 100 |
| Capsul (20% aqueous solution) | 52 | | | |
| Glucidex^{®} 6 (20% aqueous solution) Litesse^{®} Ultra | | 52 | | |
| (20% aqueous solution) | | | 52 | |
| Purity Gum 2000 (20% aqueous solution) | | | | 52 |
| Citric acid | to pH = 5 | to pH = 5 | to pH = 5 | to pH = 5 |

| | **POWDER CHARACTERISTICS** | | | |
|---|---|---|---|---|
| Yield of spray-dried powder | 80% | 70% | 70% | 80% |
| Weight of encapsulated fragrance | 60% | 60% | 60% | 60% |
| Free fragrance | <1% | <1% | <1% | <1% |
| Particle size of powder | 35 µm | 81 µm | 101 µm | 28 µm |
| Deposits on walls/Removal | no deposits, no easy to rinse with water | deposits, easy to rinse with water | no deposits, easy to rinse with water | no deposits, easy to rinse with water |
| Amine smell | none | none | none | none |

The addition of Capsul^{®} and Purity Gum to the aqueous slurries resulted in spray-dried powders with a more homogeneous particle size distribution and a much smaller average particle size that those of the powders obtained by adding Litesse^{®} Ultra or Glucidex^{®} to the aqueous dispersions of microcapsules.

### Analytical measurements:

The amount of encapsulated fragrance in the dry powder was determined by hydrodistillation.

Particle size of dry powder was determined by laser diffraction from a sample of the unsieved product. Particle size of the capsule slurry and the powder dispersed in water was determined by laser diffraction with Malvern particle size analyser. The results of the volume weighted average are presented as D[4,3] in microns.

Explosivity was determined by a 20 liter Sphere Apparatus. The 20 liter sphere monitors the evolution of the explosion pressure of fuel/air mixtures in a closed vessel (under vacuum, chemical ignition source of 10 kJ) with time, over a range of product concentrations. This enables determination of the maximum explosion pressure, Pmax, the dust explosion violence, Kst, and the dust explosion hazard class, the ST-class, according to VDI-3673.

The amount of external fragrance on the spray-dried microcapsules was determined by mild extraction of the powder with isooctane for 10 minutes. The centrifuged solvent was injected into a GC-MS spectrometer and the amount of fragrance quantified by comparison to a reference of a 1% by weight solution of the fragrance in isooctane.

### Example 3

### Preparation of melamine-formaldehyde microcapsule powders

Similar tests to those described in Example 2 were carried out with the ingredients summarized in Table II, in the amounts indicated, using the microcapsule slurries prepared in Example 1.

The dispersions obtained were then spray-dried at Büchi Spray Dryer Model B 290.

The temperature of the incoming air in the spray dryer was 185°C - 190°C, the temperature of the outcoming air 95°C - 105°C. A biphase spray nozzle was used with a nozzle diameter of 0.7 mm.

The results of these tests are summarized in Table II.

**Table II**

| | **SAMPLE / Parts by weight** | | | | | | | |
|---|---|---|---|---|---|---|---|---|
| | **Comparative** | | | | | **Invention** | | |

| **Ingredients** | **E** | **F** | **G** | **H** | **I** | **K** | **L** | **M** |
|---|---|---|---|---|---|---|---|---|
| Example 1a) slurry | 100 | | | | | 97 | | |
| Example 1b) slurry | | | | 100 | | | | |
| Example 1c) slurry | | | | | 100 | | | |
| Example 1d) slurry | | 100 | 100 | | | | 97 | 97 |
| Citric acid | to pH 6 | to pH 6 | to pH 6 | to pH 5 | to pH 5 | to pH 5 | to pH 5 | to pH 5 |
| Capsul^{®} (20% aqueous solution) | | | | | | 15 | 15 | 15 |
| K₃Citrate | | | | | | | | 2.5 |
| Yield of spray-dried powder | 10% | 10% | 10% | 10% | 10% | 80% | 80% | 30% |

| | **POWDER CHARACTERISTICS** | | | | | | | |
|---|---|---|---|---|---|---|---|---|
| Encapsulated fragrance weight | n.d. | n.d. | n.d. | n.d. | n.d. | 64% | 59% | 59% |
| Free fragrance weight | n.d | n.d. | n.d. | n.d. | n.d. | 0.08% | 4% | 4% |
| Comments | powder sticks on reactor walls | powder sticks on reactor walls | powder sticks on reactor walls | powder sticks on reactor walls | powder sticks k on reactor walls | no deposit easy to rinse with water | no deposit easy to rinse with water | K₃Citrate causes gel formation but easy to rinse with water |
| amine smell | strong | strong | none | none | none | none | none | none |
| | | | | | | 23 µm | 43 µm | 27 µm i |
| Particle size of powder dispersed in water | | | | | | 2 - 3 µm | | |
| Explosion violence, Kst | n.d. | n.d. | n.d. | n.d. | n.d. | 131 bar m/s | 224 bar m/s | 93 bar m/s |
| Explosion hazard class | n.d. | n.d. | n.d. | n.d. | n.d. | ST-1 | ST-2 | ST-1 |

| | | | | | | | | |
|---|---|---|---|---|---|---|---|---|
| n.d. - not determined | | | | | | | | |

### Example 4

### Preparation of a Baby Oil Gel containing spray-dried fragranced aminoplast capsules

A baby oil gel was prepared by admixture, in a generally known manner, of the ingredients cited in Table III below, in the proportions indicated.

**Table III**

| Ingredient | Weight % |
|---|---|
| **Part A** | |
| Versagel^{®} M750 (from Penreco) | 74.60 |
| Isopropyl Isostearate | 13.00 |
| Propylene Glycol Isoceteth-3 Acetate | 10.00 |
| **Part B** | |
| Isopropyl Isostearate | 2.00 |
| Propylparaben | 0.10 |
| **Part C** | |
| Spray-dried microcapsules of Example 3 L, | 0.30 |
| containing limonene | |

The part A ingredients were mixed at 70°C until reaching a uniform mixture. Part B was heated to 60°C until clear. Premixed part B was then added to part A and they were mixed together until obtaining a homogeneous mixture that was then cooled down to 45°C before adding part C to it.

The microcapsules remained homogeneously dispersed in the above formula during a 3 months storage at ambient temperature (around 25°C).

### Example 5

### Preparation of a Non-Aqueous (water-free) Liquid Fabric Softener containing spray-dried fragranced aminoplast capsules

An anhydrous liquid fabric softener was prepared by admixture, in a generally known manner, in the order and proportions indicated in Table IV below, of the cited ingredients, using the powder microcapsules of Sample L described in Table II of Example 3.

**Table IV**

| Ingredient | Weight % |
|---|---|
| Polydimethylsiloxane | 19.00 |
| Trioleate Glycerol | 15.00 |
| Sunflower Oil | 60.70 |
| Free Perfume | 5.00 |
| Spray-dried microcapsules of Example 3 L, containing limonene | 0.30 |

The above liquid softener formulation was packaged into a polyvinyl alcohol sachet having film thickness of about 0.3 mm. The sachets containing the above formula remained stable during 3 months storage at room temperature and they dissolved rapidly within one to two minutes of starting a wash cycle.

## Claims

1. A process for the preparation of powder products, wherein an aqueous dispersion or slurry of aminoplast microcapsules comprising an encapsulated oil is spray-dried into a powder in a generally known manner, the process being **characterized in that** it comprises the following steps, prior to the spray-drying step:
a) addition to said dispersion or slurry of a modified polydextrose derivative, preferably dioctenylsuccinated starch or hydrogen octenylbutanedioate starch, in an appropriate amount; and
b) addition of an acid to the dispersion, or slurry, in an amount sufficient to bring the pH of the dispersion, or slurry, thus obtained to a value comprised between 4 and 6.

2. A process according to claim 1, wherein the acid is selected from the group consisting of citric acid, hydrochloric acid and sulfonic acid.

3. A process according to claim 1, wherein the aqueous dispersion or slurry contains a flame retardant agent.

4. A process according to any one of claims 1 to 3, wherein the aqueous dispersion, or slurry, contains from 0.5 to 50% by weight of microcapsules, relative to the total weight of the dispersion or slurry.

5. A process according to any one of the preceding claims, wherein the aminoplast capsules are melamine-formaldehyde based microcapsules.

6. A process according to claim 5, wherein the modified polydextrose derivative is added to the dispersion, or slurry, in an amount comprised between 1 and 10%, and more preferably between 1 and 3% by weight, relative to the total weight.

7. A process according to claim 1 or 5, wherein the oil encapsulated is a fragrance or ingredient or composition, an antibacterial or malodor counteracting agent.

8. A process according to claim 5, wherein the encapsulated oil is a fragrance comprising volatile ingredients with flash points lower than 50°C.

9. A process according to claim 1, wherein the produced powder comprises from 1 to 90% by weight of encapsulated oil, relative to the weight of powder.

10. A spray dried powder susceptible of being obtained by the process according to any one of claims 1 to 9.

11. A spray dried powder according to claim 10, comprising from 1 to 90% by weight of a fragrance ingredient composition, relative to the weight of powder.

12. A spray dried powder according to claim 11, wherein said fragrance contains volatile ingredients with flash points lower than 50°C.

13. A consumer product comprising a spray dried powder according to claim 10, in the form of a water-free liquid detergent or fabric softener, a hair or body oil or gel, a hair or body spray based on cyclomethicone, a powder detergent or a fabric conditioning sheet.

14. A consumer product according to claim 13, wherein the amount of said powder is comprised between 0.1 and 10% by weight, relative to the weight of consumer product.

15. A method to improve the properties, or attractiveness, of a surface such as skin, hair, tile, glass, plastic, concrete or textile surface, which comprises applying to said surface a consumer product according to claim 13 or 14.

## Patentansprüche

1. Verfahren für die Herstellung von Pulverprodukten, wobei eine wässerige Dispersion oder Aufschlämmung von Aminoplastmikrokapseln, umfassend ein eingekapseltes Öl, in einer allgemein bekannten Weise zu einem Pulver sprühgetrocknet wird, wobei das Verfahren **dadurch gekennzeichnet ist, daß** es vor dem Sprühtrocknungsschritt die folgenden Schritte umfaßt:
a) Zugabe zu der Dispersion oder Aufschlämmung eines modifizierten Polydextrose-Derivats, vorzugsweise dioctenylsuccinierte Stärke oder Hydrogenoctenylbutandioat-Stärke, in einer passenden Menge; und
b) Zugabe einer Säure zu der Dispersion oder Aufschlämmung in einer Menge, ausreichend, um den pH der so erhaltenen Dispersion oder Aufschlämmung auf einen Wert zwischen 4 und 6 zu bringen.

2. Verfahren gemäß Anspruch 1, wobei die Säure aus der Gruppe, bestehend aus Citronensäure, Chlorwasserstoffsäure und Sulfonsäure, ausgewählt ist.

3. Verfahren gemäß Anspruch 1, wobei die wässerige Dispersion oder Aufschlämmung ein flammhemmendes Mittel enthält.

4. Verfahren gemäß einem der Ansprüche 1 bis 3, wobei die wässerige Dispersion oder Aufschlämmung von 0,5 bis 50 Gew.-%, relativ zu dem Gesamtgewicht der Dispersion oder Aufschlämmung, Mikrokapseln enthält.

5. Verfahren gemäß einem der vorhergehenden Ansprüche, wobei die Aminoplastkapseln Mikrokapseln auf Melamin-Formaldehyd-Basis sind.

6. Verfahren gemäß Anspruch 5, wobei das modifizierte Polydextrose-Derivat zu der Dispersion oder Aufschlämmung in einem Anteil zwischen 1 und 10 Gew.-% und stärker bevorzugt zwischen 1 und 3 Gew.-%, relativ zu dem Gesamtgewicht, hinzugefügt wird.

7. Verfahren gemäß Anspruch 1 oder 5, wobei das eingekapselte Öl ein Duftstoff -Bestandteil oder eine Duftstoff -Zusammensetzung, ein antibakterielles oder ein üblem Geruch entgegenwirkendes Mittel ist.

8. Verfahren gemäß Anspruch 5, wobei das eingekapselte Öl ein Duftstoff, umfassend flüchtige Bestandteile mit Entzündungspunkten niedriger als 50°C, ist.

9. Verfahren gemäß Anspruch 1, wobei das erzeugte Pulver von 1 bis 90 Gew.-%, relativ zu dem Gewicht des Pulvers, eingekapseltes Öl umfaßt.

10. Sprühgetrocknetes Pulver, das sich durch das Verfahren gemäß einem der Ansprüche 1 bis 9 erhalten läßt.

11. Sprühgetrocknetes Pulver gemäß Anspruch 10, umfassend von 1 bis 90 Gew.-%, relativ zu dem Gewicht des Pulvers, eines Duftstoff -Bestandteils oder einer Duftstoff - Zusammensetzung.

12. Sprühgetrocknetes Pulver gemäß Anspruch 11, wobei der Duftstoff flüchtige Bestandteile mit Entzündungspunkten niedriger als 50°C enthält.

13. Verbraucherprodukt, umfassend ein sprühgetrocknetes Pulver gemäß Anspruch 10 in der Form eines wasserfreien flüssigen Detergens oder Weichspülmittels, eines Haar- oder Körperöls oder -gels, eines Haar- oder Körpersprays, basierend auf Cyclomethicon, eines Pulverdetergens oder eines Gewebekonditionierungsblattes.

14. Verbraucherprodukt gemäß Anspruch 13, wobei der Anteil des Pulvers zwischen 0,1 und 10 Gew.-%, relativ zu dem Gewicht des Verbraucherprodukts, liegt.

15. Verfahren zum Verbessern der Eigenschaften, oder der Attraktivität einer Oberfläche wie beispielsweise Haut-, Haar-, Fliesen-, Glas-, Kunststoff-, Beton- oder Textiloberfläche, welches Aufbringen eines Verbraucherprodukts gemäß Anspruch 13 oder 14 auf die Oberfläche umfaßt.

## Revendications

1. Procédé de préparation de produits en poudre, dans lequel une dispersion ou suspension aqueuse de microcapsules d'aminoplaste comprenant une huile encapsulée est séchée par atomisation pour donner une poudre d'une manière généralement connue, ce procédé étant **caractérisé en ce qu'**il comprend les étapes suivantes, avant l'étape de séchage par atomisation:
a) addition à ladite dispersion ou suspension d'un dérivé de polydextrose modifié, de préférence un amidon fonctionnalisé avec un diocténylsuccinate ou un amidon fonctionnalisé avec un hydrogénoocténylbutanedioate, en une quantité appropriée; et
b) addition d'un acide à la dispersion ou suspension, en une quantité suffisante pour amener le pH de la dispersion ou suspension ainsi obtenue à une valeur comprise entre 4 et 6.

2. Procédé selon la revendication 1, dans lequel l'acide est choisi dans le groupe constitué de l'acide citrique, de l'acide chlorhydrique et de l'acide sulfonique.

3. Procédé selon la revendication 1, dans lequel la dispersion ou suspension aqueuse contient un agent ignifuge.

4. Procédé selon l'une quelconque des revendications 1 à 3, dans lequel la dispersion ou suspension aqueuse contient de 0,5 à 50% en poids de microcapsules, par rapport au poids total de la dispersion ou suspension.

5. Procédé selon l'une quelconque des revendications précédentes, dans lequel les capsules d'aminoplaste sont des microcapsules à base de mélamine-formaldéhyde.

6. Procédé selon la revendication 5, dans lequel le dérivé de polydextrose modifié est ajouté à la dispersion ou suspension en une quantité comprise entre 1 et 10%, et plus préférentiellement entre 1 et 3% en poids, par rapport au poids total.

7. Procédé selon la revendication 1 ou 5, dans lequel l'huile encapsulée est un ingrédient de parfum ou une composition de parfum, un agent antibactérien ou un agent de lutte contre les mauvaises odeurs.

8. Procédé selon la revendication 5, dans lequel l'huile encapsulée est un parfum comprenant des ingrédients volatils ayant des points d'éclair inférieurs à 50°C.

9. Procédé selon la revendication 1, dans lequel la poudre produite comprend de 1 à 90% en poids d'huile encapsulée, par rapport au poids de poudre.

10. Poudre séchée par atomisation susceptible d'être obtenue par le procédé selon l'une quelconque des revendications 1 à 9.

11. Poudre séchée par atomisation selon la revendication 10, comprenant de 1 à 90% en poids d'un ingrédient de parfum ou d'une composition de parfum, par rapport au poids de poudre.

12. Poudre séchée par atomisation selon la revendication 11, dans laquelle ledit parfum contient des ingrédients volatils ayant des points d'éclair inférieurs à 50°C.

13. Produit de consommation comprenant une poudre séchée par atomisation selon la revendication 10, sous forme de détergent liquide exempt d'eau ou d'adoucissant pour textile, d'huile ou de gel capillaire ou corporel, de pulvérisation capillaire ou corporelle à base de cyclométhicone, de détergent en poudre ou de feuille de conditionnement de textile.

14. Produit de consommation selon la revendication 13, dans lequel la quantité de ladite poudre est comprise entre 0,1 et 10% en poids, par rapport au poids du produit de consommation.

15. Procédé pour améliorer les propriétés, ou l'attractivité, d'une surface telle que la surface de la peau, des cheveux, des carreaux, du verre, du plastique, du béton ou des textiles, qui consiste à appliquer sur ladite surface un produit de consommation selon la revendication 13 ou 14.
